(19) Europäisches Patentamt
European Patent Office
Office européen des brevets

(11) Veröffentlichungsnummer: **0 207 396**
**B1**

(12) **EUROPÄISCHE PATENTSCHRIFT**

(45) Veröffentlichungstag der Patentschrift: **19.04.89**

(51) Int. Cl.⁴: **C 07 D 405/12**, C 08 K 5/15, C 08 K 5/34, C 09 D 7/12

(21) Anmeldenummer: **86108428.3**

(22) Anmeldetag: **20.06.86**

# (54) Tetrahydrofurancarbonsäurederivate.

(30) Priorität: **25.06.85 DE 3522678**

(43) Veröffentlichungstag der Anmeldung: **07.01.87 Patentblatt 87/2**

(45) Bekanntmachung des Hinweises auf die Patenterteilung: **19.04.89 Patentblatt 89/16**

(84) Benannte Vertragsstaaten: **CH DE FR GB IT LI**

(56) Entgegenhaltungen:
**EP-A-0 103 115**
**DE-A-1 929 928**
**DE-A-2 040 975**
**DE-A-2 258 752**
**DE-A-2 349 962**
**DE-A-2 623 422**
**DE-A-2 735 051**
**DE-A-3 117 389**
**DE-A-3 345 376**

**CHEMICAL ABSTRACTS, Band 88, no. 2, 9. Januar 1978, Columbus, Ohio, USA, A.A. IVANOV; A.E. PRIMELLES; F.A. GIL "Synthesis and some characteristics of 2,5-furandicarboxylic acid polymers", p. 3, Zusammenfassung no. 7 444y**
**CHEMICAL ABSTRACTS, Band 89, no. 14, 2. Oktober 1978, Columbus, Ohio, USA, J.A. MOORE; J.E. KELLY "Polyesters derived from furan and**

(73) Patentinhaber: **BASF Aktiengesellschaft, Carl-Bosch- Strasse 38, D-6700 Ludwigshafen (DE)**

(72) Erfinder: **Helwig, Reinhard, Dr., Brüsseler Ring 4, D-6700 Ludwigshafen (DE)**
Erfinder: **Neumann, Peter, Dr., Franz- Schubert-Strasse 1, D-6908 Wiesloch (DE)**
Erfinder: **Aumüller, Alexander, Dr., Brechlochstrasse 9, D-6700 Ludwigshafen (DE)**
Erfinder: **Trauth, Hubert, Milanstrasse 6, D-6724 Dudenhofen (DE)**

(56) Entgegenhaltungen: (Fortsetzung)
**tetrahydrofuran nuclei", p. 6, Zusammenfassung no. 110 483e**

EP 0 207 396 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

## Beschreibung

Es ist bereits bekannt, daß 2,2,6,6-Tetraalkylpiperidinderivate ausgezeichnete Lichtschutzmittel für organische Polymere sind. Unter ihnen sind auch verschiedene Carbonsäurederivate von Sauerstoffheterocyclen mit fünf Ringgliedern beschrieben worden. Hierbei handelt es sich um Furancarbonsäurederivate.

In allgemeiner Form sind sauerstoffhaltige heterocyclische Carbonsäureester von 2,2,6,6-Tetraalkylpiperidinen in den DE-A-2 258 752 und 1 929 928, solche von 2,2,3,6,6-Pentaalkyl- und 1,2,2,3,6,6-Hexaalkylpiperidinen in der DE-A-2 623 422 beansprucht. Sauerstoffhaltige heterocyclische Carbonsäureamide von 1,2,2,6,6-Pentaalkylpiperidinen umfaßt in allgemeiner Form die DE-A-2 349 962.

Schließlich beschreibt die EP-A-111 448 Amide der Tetrahydrofuran-2,5-dicarbonsäure von 1,2,2,6,6-Penta- und 1,2,2,3,6,6-Hexaalkylpiperidinen, die im Molekül eine phenolische Gruppierung enthalten, als Bestandteil von farbphotographischem Aufzeichnungsmaterial.

Aufgabe der vorliegenden Erfindung war es weitere Mittel, welche Polymere, insbesondere Polyolefine ausgezeichnet stabilisieren und die mit den zu schützenden Polymeren gut verträglich sind, bereitzustellen.

Die Erfindung betrifft nun Tetrahydrofurancarbonsäurederivate der allgemeinen Formel I

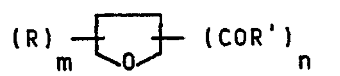 (I),

worin

R unabhängig voneinander $C_1$- bis $C_4$-Alkyl, Cyclohexyl oder Phenyl,
m 0 bis 3,
n 1 oder 2 und
R' ein Rest der Formel

$-OR^2$ , 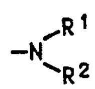 oder 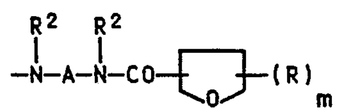

sind,

A $C_2$- bis $C_{12}$-Alkylen, $-(CH_2-)_2-O-(CH_2-)_2$, $-(CH_2-)_3-O-(CH_2-)_3$, $-(CH_2-)_3-O-(CH_2-)_2-O-(-CH_2-)_3$,

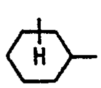 , 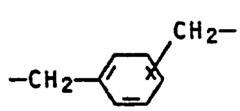 , 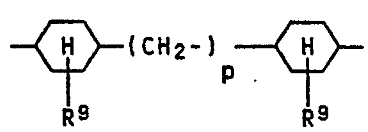

oder

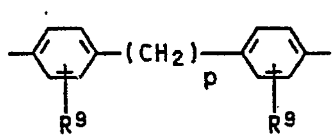

$R^1$ Wasserstoff $C_2$- bis $C_6$-Alkenyl, gegebenenfalls bis zu 3 mal durch Sauerstoff unterbrochenes $C_1$- bis $C_{12}$-Alkyl, gegebenenfalls substituiertes Phenylalkyl oder $C_5$- bis $C_7$-Cycloalkyl und
$R_2$ ein Rest der Formel

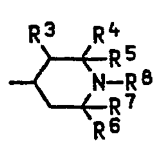

sind, in dem

$R^3$ Wasserstoff oder Methyl,
$R^4$ bis $R^7$ Methyl oder Ethyl,

$R^6$ Wasserstoff, $C_1$- bis $C_8$-Alkyl, $C_3$- bis $C_8$-Alkenyl, $C_2$- bis $C_4$-Hydroxyalkyl oder Aralkyl,
$R^9$ Wasserstoff oder Methyl und
p 0, 1 oder 2 bedeuten,

sowie Salze der Verbindungen (I).

Alkylreste für R sind im Rahmen der allgemeinen Definition die geradkettigen oder verzweigten Gruppen $C_nH_{2n+1}$. Bevorzugt sind die unverzweigten Reste. Im einzelnen seien beispielsweise genannt:

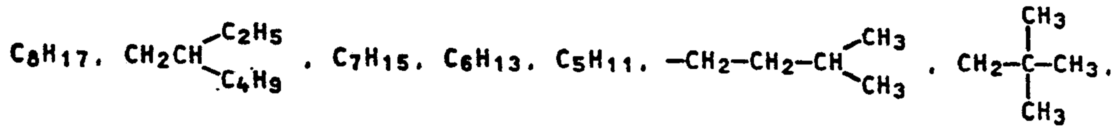

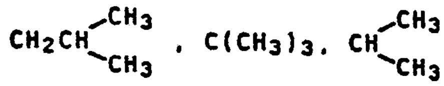 vorzugsweise $CH_3$, $C_2H_5$, $C_3H_7$ oder $C_4H_9$.

Ethersauerstoff enthaltende Alkylgruppen $R^1$ sind beispielsweise:

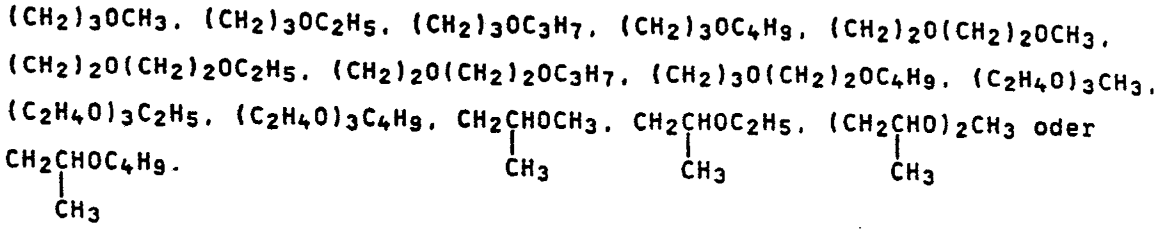

Als Alkenylgruppen sind z. B. Vinyl, Allyl, Methallyl oder 2-Butenyl zu nennen.

Phenylalkylreste $R^1$ sind z. B. $(CH_2)_qC_6H_5$, $(CH_2)_qC_6H_4OCH_3$, $(CH_2)_qC_6H_3(OCH_3)_2$, $(CH_2)_qC_6H_4CH_3$, $(CH_2)_qC_6H_4Cl$, wobei q 1 bis 4 ist.

Cycloalkylreste für $R^1$ sind z. B. Cyclopentyl, -hexyl oder -heptyl.

Alkenyl-, Hydroxyalkyl- und Aralykreste für $R^8$ sind beispielsweise:

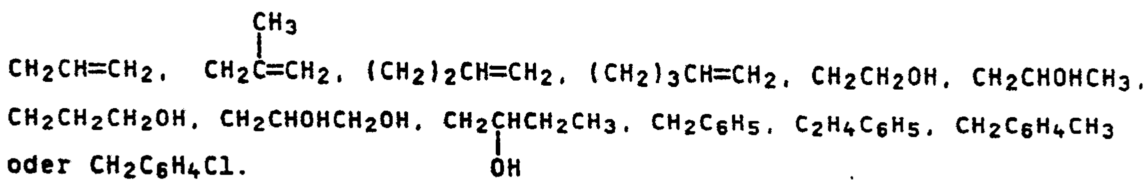

Brückenglieder A sind $C_2$- bis $C_{12}$-Alkylengruppen, $(CH_2)_2O(CH_2)_2$, $(CH_2)_3O(CH_2)_3$ oder $(CH_2)_3O(CH_2)_2O(CH_2)_3$, sowie Reste der Formeln

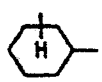 , 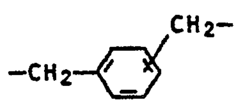 , 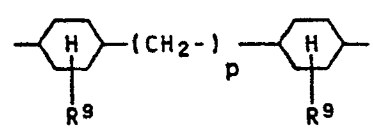

oder 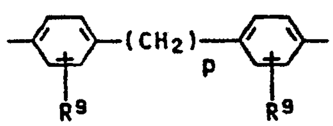

wobei $R^9$ Wasserstoff oder Methyl und p 0, 1 oder 2 sind.

Einzelne Alkylenreste sind z. B.: $C_2H_4$, $(CH_2)_3$, $(CH_2)_4$, $(CH_2)_6$.

Bevorzugte Verbindungen der Formel I haben als

R Wasserstoff oder Methyl, als
$R^3$ Wasserstoff und als
$R^4$ bis $R^7$ Methyl.

Die erfindungsgemäßen Verbindungen können nach allgemein bekannten chemischen Verfahren hergestellt werden. So besteht beispielsweise ein wichtiger Zugang zu den erfindungsgemäßen Verbindungen in der katalytischen Hydrierung entsprechender Furanderivate. Derartige Furanderivate sind bereits bekannt. Z. B. erwähnen die DE-PS-1 929 928 und 2 258 752 Piperidinylester und die DE-PS-2 040 975 und DE-PS-2 349 962 Piperidinylamide der Furan-2-carbonsäure. Die Patentanmeldung P 3 345 376.4 umfaßt Piperidinylester und -amide verschiedener Furan-3-carbonsäuren. In analoger Weise können auch andere, bisher nicht im Einzelnen beschriebene Verbindungen, z. B. Derivate der Furan-2,5-dicarbonsäure, der Furan-3,4-dicarbonsäure, der 2,5-Dimethylfuran-3,4-dicarbonsäure usw. hergestellt werden. Alle derartigen Verbindungen lassen sich unter geeigneten Bedingungen problemlos katalytisch hydrieren, wobei, je nach Substitution des Furanringes, verschiedene Stereoisomere entstehen können, was aber der stabilisierenden Wirksamkeit nicht abträglich ist.

Ein anderer Weg zu den erfindungsgemäßen Verbindungen geht von den Tetrahydrofurancarbonsäuremethyl- oder ethylestern bzw. den entsprechenden Säurechloriden, soweit solche bekannt sind, aus. Die Tetrahydrofurancarbonsäurealkylester können z. B. mit den gewünschten 4-Hydroxipiperidinen in Gegenwart katalytisch wirkender Verbindungen, wie Alkalialkoholaten oder Titantetrabutylat, umgeestert werden, wobei der entsprechende niedere Alkohol destillativ entfernt wird. Die Tetrahydrofurancarbonsäurechloride können mit 4-Aminopiperidinderivaten in Gegenwart säurebindender Mittel, wie Alkalicarbonaten oder organischen Aminen, zu den entsprechenden Säureamiden umgesetzt werden.

Die Herstellung zahlreicher, als Ausgangsverbindungen benötigter Furan- bzw. Tetrahydrofurancarbonsäure- oder -dicarbonsäurederivate kann der Literatur entnommen werden. Vielfach lassen sich auch literaturbekannte Herstellverfahren problemlos auf bisher nicht beschriebene Furan- oder Tetrahydrofurancarbonsäurederivate übertragen.

Die erfindungsgemäßen Verbindungen können in Form der freien Basen oder aber auch als Salze vorliegen. Geeignete Anionen stammen z. B. von anorganischen Säuren und insbesondere organischen Carbonsäuren.

Anorganische Anionen sind z. B. Chlorid, Bromid, Sulfat, Methosulfat, Phosphat oder Rhodanid.

Carbonsäure-Anionen sind beispielsweise: Formiat, Acetat, Propionat, Hexanoat, Cyclohexanoat, Lactat, Stearat, Dodecylbenzoat, Benzoat, Acrylat, Methacrylat, Zitrat, Malonat oder Succinat sowie Anionen von Polycarbonsäuren mit bis zu 100 COOH-Gruppen.

Einzelheiten der Herstellung können den Beispielen entnommen werden.

Die erfindungsgemäßen Verbindungen eignen sich zum Stabilisieren von organischem Material, speziell von Kunststoffen, gegen den Abbau durch Licht und Wärme. Sie werden den zu stabilisierenden Kunststoffen in einer Konzentration von 0,01 bis 5 Gew.-%, vorzugsweise 0,02 bis 1 Gew.-% vor, während oder nach der Polymerbildung zugesetzt.

Zur Vermischung der erfindungsgemäßen Verbindungen mit den zu stabilisierenden Kunststoffen können alle bekannten Vorrichtungen und Methoden zum Einmischen von Stabilisierungsmitteln oder anderen Zusätzen in Polymere angewandt werden.

Die durch eine der erfindungsgemäßen Verbindungen stabilisierten Kunststoffe können gegebenenfalls noch weitere Additive enthalten, beispielsweise Antioxidantien, Lichtstabilisierungsmittel, Metalldesaktivatoren, antistatische Mittel, flammhemmende Mittel, Pigmente und Füllstoffe.

Antioxidantien und Lichtstabilisatoren, die den Kunststoffen neben den erfindungsgemäßen Verbindungen zugesetzt werden können, sind z. B. Verbindungen auf der Basis sterisch gehinderter Phenole oder Schwefel oder Phosphor enthaltende Costabilisatoren.

Als derartige phenolische Antioxidationsmittel seien beispielsweise 2,6-Di-tert.-butyl-4-methylphenol, n-Octadecyl-β-(3,5-di-tert.-butyl-4-hydroxyphenyl)-propionat, 1,1,3-Tris-(2-methyl-4-hydroxy-5-tert.-butylphenyl)-butan, 1,3,5-Trimethyl-2,4,6-tris-(3,5-di-tert.-butyl-4-hydroxybenzyl)-benzol, 1,3,5-Tris-(3,5-di-tert.-butyl-4-hydroxybenzyl)-isocyanurat, 1,3,5-Tris-[β-(3,5-di-tert.-butyl-4-hydroxyphenyl)-propionyloxyethyl]-isocyanurat, 1,3,5-Tris-(2,6-di-methyl-3-hydroxy-4-tert.-butylbenzyl)-isocyanurat, Pentaerythrit-tetrakis-[β-(3,5-di-tert.-butyl-4-hydroxyphenyl)-propionat] etc. erwähnt.

Als phosphorhaltige Antioxidantien seien beispielsweise Tris-(nonylphenyl)-phosphit, Distearylpentaerythritdiphosphit, Tris-(2,4-di-tert.-butylphenyl)-phosphit, Tris-(2-tert.-butyl-4-methylphenyl)-phosphit, Bis-(2,4-di-tert.-butylphenyl)-pentaerythritdiphosphit, Tetrakis-(2,4-di-tert.-butylphenyl)-4,4'-biphenylendiphosphit etc. erwähnt.

Als Schwefel enthaltende Antioxidationsmittel seien beispielsweise Dilaurylthiodipropionat, Dimyristylthiodipropionat, Distearylthiodipropionat, Pentaerythrittetrakis-(β-laurylthiopropionat), Pentyerythrittetrakis-(β-hexylthiopropionat) etc. erwähnt.

Weitere Antioxidantien und Lichtstabilisatoren, die zusammen mit den erfindungsgemäßen Verbindungen verwendet werden können, sind z. B. 2-(2'-Hydroxyphenyl)-benztriazole, 2-Hydroxybenzophenone, Arylester von Hydroxybenzoesäuren, α-Cyanozimtsäurederivate, Nickelverbindungen oder Oxalsäuredianilide.

Als organische Polymere, die durch die erfindungsgemäßen Verbindungen stabilisiert werden können, seien beispielsweise genannt:

Polymere von Mono- und Diolefinen, wie z. B. Polyethylen niedriger oder hoher Dichte, lineares Polyethylen niedriger Dichte, Polypropylen, Polyisobutylen, Polybuten-1, Polymethylpenten-1, Polyisopren, Polybutadien sowie Copolymerisate von Mono- und Diolefinen oder Mischungen der genannten Polymeren;

Copolymerisate von Mono- oder Diolefinen mit anderen Vinylmonomeren wie z. B. Ethylen-Alkylacrylat-Copolymere, Ethylen-Alkylmethacrylat-Copolymere, Ethylen-Vinylacetat-Copolymere oder Ethylen-Acrylsäure-Copolymere;

Polystyrol;

Copolymere von Styrol oder α-Methylstyrol mit Dienen oder Acrylderivaten, wie z. B. Styrol-Butadien, Styrol-Acrylnitril, Styrol-Ethylmethacrylat, Styrol-Butadien-Ethylacrylat, Styrol-Acrylnitril-Methacrylat;

ABS-, MBS- oder ähnliche Polymere;

Halogenhaltige Polymere, wie z. B. Polyvinylchlorid, Polyvinylfluorid, Polyvinylidenfluorid sowie deren Copolymere;

Polymere die sich von α,β-ungesättigten Säuren und deren Derivaten ableiten, wie Polyacrylate und Polymethacrylate, Polyacrylamide und Polyacrylnitrile;

Polymere, die sich von ungesättigten Alkoholen und Aminen bzw. deren Acrylderivaten oder Acetalen ableiten, wie Polyvinylalkohol oder Polyvinylacetat;

Polyurethane, Polyamide, Polyharnstoffe, Polyester, Polycarbonate, Polysulfone und Polyethersulfone.

Weitere organische Polymere, die mit den erfindungsgemäßen Verbindungen stabilisiert werden können, stellen Industrielackierungen dar. Unter diesen sind Einbrennlackierungen, unter diesen wiederum Fahrzeuglackierungen, vorzugsweise Zweischichtlackierungen, besonders hervorzuheben.

Auch hier können zusätzlich die bereits aufgeführten Antioxidantien und Lichtschutzmittel verwendet werden.

Die erfindungsgemäßen Verbindungen können, soweit sie fest sind, in fester oder gelöster Form, oder, wenn sie flüssig sind, in Substanz dem Lack zugesetzt werden. Ihre gute Löslichkeit in Lacksystemen ist dabei von besonderem Vorteil.

Bevorzugt ist die Verwendung der erfindungsgemäßen Verbindungen in Polyolefinen, vorzugsweise Ethylen- und Propylenpolymerisaten sowie in Lacksystemen.

Im Hinblick auf den Stand der Technik war es überraschend und nicht vorhersehbar, daß die gewünschten Verbindungen der Formel I und II eine ausgezeichnete stabilisierende Wirkung auf Polymere ausüben und zudem eine hervorragende Verträglichkeit gegenüber Polymeren, speziell Polyolefinen, aufweisen würden.

**Beispiel 1**

2,5-Dimethyltetrahydrofuran-3-carbonsäure-2,2,6,6-tetramethyl-4-piperidinylester.

**Methode A**

In einen 0,3 l-Hydrierautoklaven werden 15 g 2,5-Dimethylfuran-3-carbonsäure-2,3,6,6-tetramethyl-4-piperidinylester, 150 ml Methanol und 3 g Raney-Nickel gefüllt und bei 150° C und 160 bar bis zur Druckkonstanz hydriert (ca. 5 Stunden). Dann wird vom Raney-Nickel abfiltriert und eingeengt. Die GC-MS-Analyse ergibt zwei isomere Hauptprodukte im gaschromatographischen Verhältnis von etwa 12 : 86. Durch Vakuumdestillation erhält man 12 g Produkt vom Sdp. 120 . 126° C/0,5 mbar als farblose Öl.

**Methode B**

In einen 0,3 l-Hydrierautoklaven werden 80 g 2,5-Dimethylfuran-3-carbonsäuremethylester, 80 ml Methanol und 5 g Raney-Nickel gefüllt und bei 150° C und 160 bar bis zur Druckkonstanz hydriert. Dann wird Raney-Nickel abfiltriert, eingeengt und im Vakuum destilliert. Man erhält 41 g Produkt vom Sdp. 28 - 30° C/0,1 mbar als farblose Flüssigkeit.

31,6 g des 2,5-Dimethyltetrahydrofuran-3-carbonsäuremethylesters werden mit 31,4 g 2,2,6,6-Tetramethyl-4-piperidinol und 2 g Tetrabutyl-ortho-titanat mit absteigendem Kühler 7 Stunden auf 200° C erhitzt. Nach dem Abkühlen wird der Rückstand in Essigester gelöst, mit 5 %-iger Sodalösung und Wasser gewaschen, getrocknet und eingeengt. Durch Vakuumdestillation erhält man das Produkt als farbloses Öl vom Sdp. 120 - 126° C/0,5 mbar.

**Beispiel 2**

Tetrahydrofuran-2-carbonsäure-2,2,6,6-tetramethyl-4-piperidinylester

In einen 0,3 l-Hydrierautoklaven werden 15 g Furan-2-carbonsäure-2,2,6,6-tetramethyl-4-piperidinylester, 150 ml Methanol und 3 g Raney-Nickel eingefüllt und bei 100° C und 100 bar bis zur Druckkonstanz hydriert (ca. 6 Stunden). Nach Abfiltrieren des Raney-Nickels und Einengen erhält man durch Vakuumdestillation 11,5 g eines

farblosen Öls vom Sdp. 118 - 122° C/1,3 mbar.

**Beispiel 3**

2,4-Dimethyltetrahydrofuran-3-carbonsäure-2,2,6,6-tetramethyl-4-piperidinylester

Durch Hydrierung von 2,4-Dimethylfuran-3-carbonsäure-2,2,6,6-tetramethyl-4-piperidinylester analog der Vorschrift von Beispiel 1 A erhält man ein farbloses Öl vom Sdp. 114° C/0,1 mbar (2 Isomere im Verhältnis 10 : 90).

**Beispiel 4**

2,5-Dimethyltetrahydrofuran-3-carbonsäure-N-butyl-N-(2,2,6,6-tetramethyl-4-piperidinyl)-amid

Durch Hydrierung von 2,5-Dimethylfuran-3-carbonsäure-N-butyl-N-(2,2,6,6-tetramethyl-4-piperidyl)-amid analog der Vorschrift von Beispiel 1 A erhält man das Produkt (2 Isomere im gaschromatographischen Verhältnis 5 : 95) als farblose viskose Masse.

**Beispiel 5**

Salz aus 2,5-Dimethyltetrahydrofuran-3-carbonsäure-N-butyl-N-(2,2,6,6-tetramethyl-4-piperidinyl)-amid und Adipinsäure

10,14 g des Carbonsäureamids aus Beispiel 4 und 2,2 g Adipinsäure werden in Methanol gerührt, bis eine klare Lösung entstanden ist. Die Lösung wird im Wasserstrahlvakuum bis zur Trockne eingeengt und der Rückstand pulverisiert. Er weist einen Schmelzpunkt von 77 - 79° C auf.

**Beispiel 6**

Bis-(N-1,6-hexamethylen-N-1,2,2,6,6-pentamethyl-4-piperidinyl)-2,5-dimethyltetrahydrofuran-3-carbonsäureamid

Durch Hydrierung von Bis-(N-1,6-hexamethylen-N-1,2,2,6,6-pentamethyl-4-piperidinyl)-2,5-dimethylfuran-3-carbonsäureamid analog der Vorschrift von Beispiel 1 A erhält man das Produkt als fast farblose viskose Masse.

**Beispiel 7**

Bis-(N-1,2-ethylen-N-1,2,2,6,6-pentamethyl-4-piperidinyl)-2,5-dimethyltetrahydrofuran-3-carbonsäureamid

Durch Hydrierung von Bis-(N-1,2-ethylen-N-1,2,2,6,6-pentamethyl-4-piperidinyl)-2,5-dimethylfuran-3-carbonsäureamid (Smp. 222 - 224° C) analog der Vorschrift von Beispiel 1 A erhält man das Produkt als viskose Masse.

**Beispiel 8**

Bis-(N-1,2-ethylen-N-2,2,6,6-tetramethyl-4-piperidinyl)-2,4-dimethyltetrahydrofuran-3-carbonsäureamid

Durch Hydrierung von Bis-(N-1,2-ethylen-N-2,2,6,6-tetramethyl-4-piperidinyl)-2,4-dimethylfuran-3-carbonsäureamid (Smp. 237 - 238° C) analog der Vorschrift von Beispiel 1 A erhält man das Produkt als viskose Masse.

**Beispiel 9**

Bis-(N-1,6-hexamethylen-N-2,2,6,6-tetramethyl-4-piperidinyl)-tetrahydrofuran-2-carbonsäureamid

Die Ausgangsverbindung, Bis-(N-1,6-hexamethylen-N-2,2,6,6-tetramethyl-4-piperidinyl)-furan-2-carbonsäureamid, wird hergestellt durch 24 stündiges Kochen von 2 Teilen 2-Furoylchlorid, 1 Teil Bis-4-(1,6-hexamethylen)-amino-2,2,6,6-tetramethylpiperidin und 1 Teil wasserfreier Soda in Toluol unter Rückfluß. Nach dem Abkühlen wird der Niederschlag getrocknet, in Wasser suspendiert und durch Zutropfen von Natronlauge ein alkalischer pH-Wert eingestellt. Durch Absaugen, Neutralwaschen und Trocknen erhält man das Produkt mit einem Smp. 110 - 112° C. Die Hydrierung erfolgt analog der Vorschrift von Beispiel 2 (Dauer ca. 9 Stunden). Man erhält das Produkt als schwach bräunliche harzige Masse.

Weitere erfindungsgemäße Verbindungen sind in der Tabelle 1 aufgeführt.

**Tabelle 1**: Verbindung der Formel 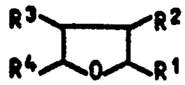

| Bsp | $R^1$ | $R^2$ | $R^3$ | $R^4$ |
|---|---|---|---|---|
| 10 | 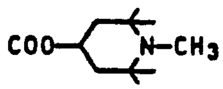 | H | H | H |
| 11 | 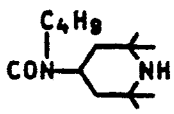 | H | H | H |
| 12 | 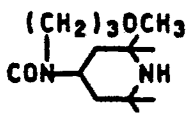 | H | H | H |
| 13 | 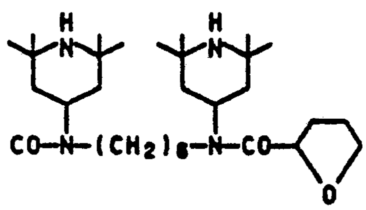 | H | H | H |
| 14 | 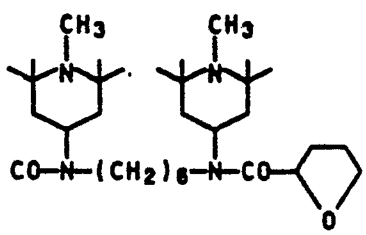 | H | H | H |
| 15 | 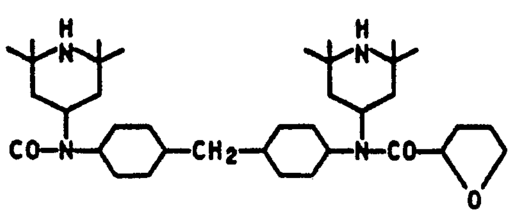 | H | H | H |
| 16 | 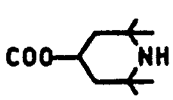 | H | H | $CH_3$ |
| 17 | 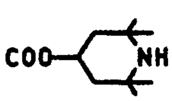 | H | H | 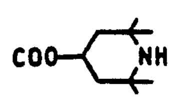 |

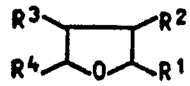

| Bsp | $R^1$ | $R^2$ | $R^3$ | $R^4$ |
|---|---|---|---|---|
| 18 | 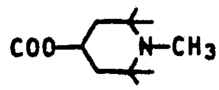 | H | H | 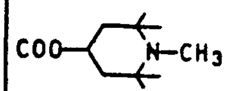 |
| 19 | 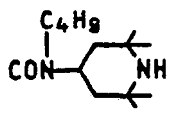 | H | H | 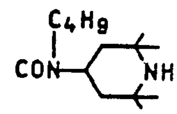 |
| 20 | H | 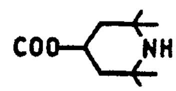 | H | H |
| 21 | H | 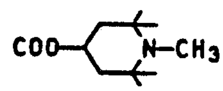 | H | H |
| 22 | H | 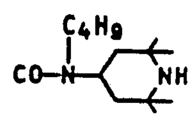 | H | H |
| 23 | $CH_3$ | 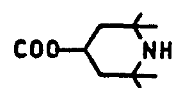 | H | H |
| 24 | $CH_3$ | 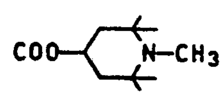 | H | H |
| 25 | $CH_3$ | 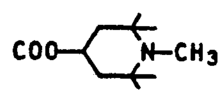 | $CH_3$ | H |
| 26 | $CH_3$ | 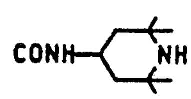 | $CH_3$ | H |
| 27 | $CH_3$ | 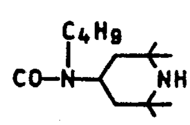 | $CH_3$ | H |

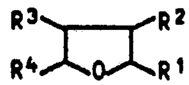

| Bsp | $R^1$ | $R^2$ | $R^3$ | $R^4$ |
|---|---|---|---|---|
| 28 | $CH_3$ | 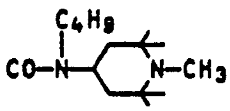 | $CH_3$ | H |
| 29 | $CH_3$ | 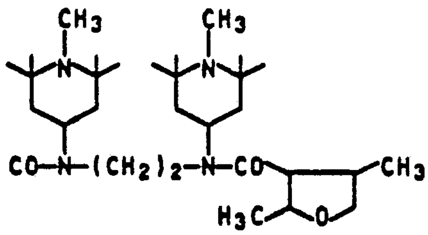 | $CH_3$ | H |
| 30 | $CH_3$ | 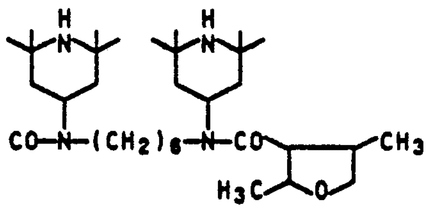 | $CH_3$ | H |
| 31 | $CH_3$ | 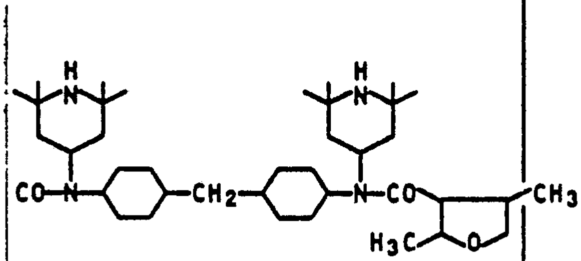 | $CH_3$ | H |
| 32 | $CH_3$ | 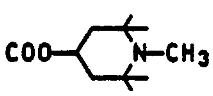 | H | $CH_3$ |
| 33 | $CH_3$ | 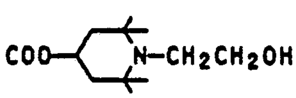 | H | $CH_3$ |
| 34 | $CH_3$ | 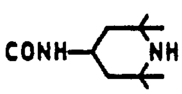 | H | $CH_3$ |

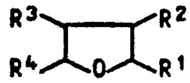

| Bsp | $R^1$ | $R^2$ | $R^3$ | $R^4$ |
|---|---|---|---|---|
| 35 | $CH_3$ | 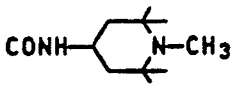 | H | $CH_3$ |
| 36 | $CH_3$ | 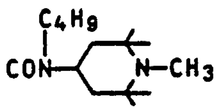 | H | $CH_3$ |
| 37 | $CH_3$ | 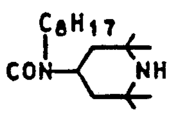 | H | $CH_3$ |
| 38 | $CH_3$ | 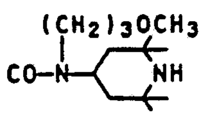 | H | $CH_3$ |
| 39 | $CH_3$ | 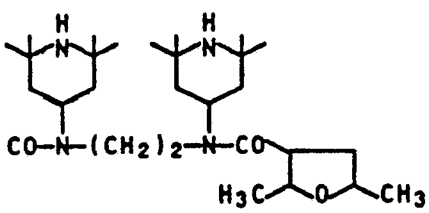 | H | $CH_3$ |
| 40 | $CH_3$ | 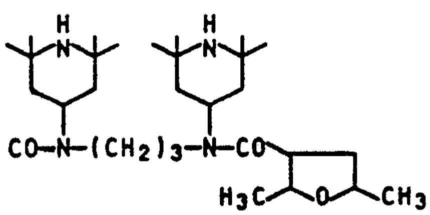 | H | $CH_3$ |
| 41 | $CH_3$ | 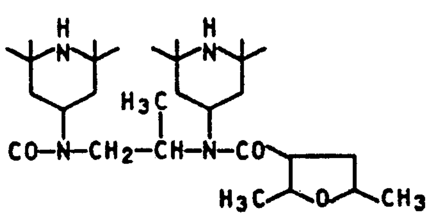 | H | $CH_3$ |

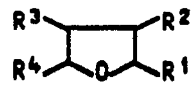

| Bsp | $R^1$ | $R^2$ | $R^3$ | $R^4$ |
|---|---|---|---|---|
| 42 | $CH_3$ | 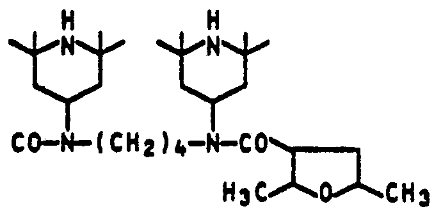 | H | $CH_3$ |
| 43 | $CH_3$ | 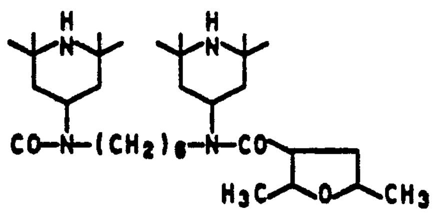 | H | $CH_3$ |
| 44 | $CH_3$ | 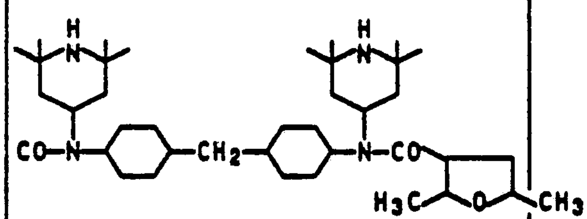 | H | $CH_3$ |
| 45 | $CH_3$ | 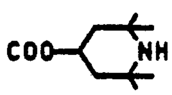 | $CH_3$ | $CH_3$ |
| 46 | $CH_3$ | 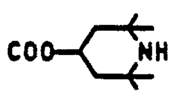 | H | $t\text{-}C_4H_9$ |
| 47 | $CH_3$ | 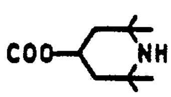 | $C_2H_5$ | H |
| 48 | $CH_3$ | 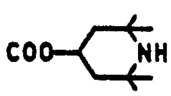 | H | 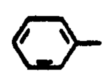 |

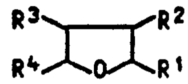

| Bsp | $R^1$ | $R^2$ | $R^3$ | $R^4$ |
|---|---|---|---|---|
| 49 | H | 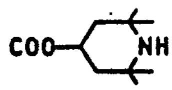 | 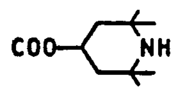 | H |
| 50 | H | 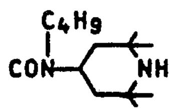 | 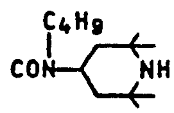 | H |
| 51 | $CH_3$ | 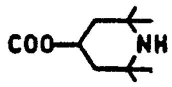 | 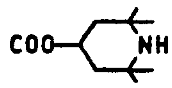 | $CH_3$ |

| | |
|---|---|
| 52 | Salz aus Verbindung Bsp. 11 und Adipinsäure |
| 53 | Salz aus Verbindung Bsp. 11 und Polyacrylsäure (ca. 28 - 42 Einheiten) |
| 54 | Salz aus Verbindung Bsp. 27 und 2,4-Dimethylglutarsäure |
| 55 | Salz aus Verbindung Bsp. 27 und Adipinsäure |
| 56 | Salz aus Verbindung Bsp. 30 und 2-Ethylhexansäure |
| 57 | Salz aus Verbindung Bsp. 4 und Polyacrylsäure (ca. 28 - 42 Einheiten) |
| 58 | Salz aus Verbindung Bsp. 4 und Polyacrylsäure (ca. 70 Einheiten) |
| 59 | Salz aus Verbindung Bsp. 43 und Adipinsäure |
| 60 | Salz aus Verbindung Bsp. 43 und 2-Ethylhexansäure |

**Beispiel 61**

N-2-(2,4-Dimethoxyphenyl)ethyl-N-2,2,6,6-tetramethyl-4-piperidinyl-2,5-dimethyl-tetrahydrofuran-3-carbonsäureamid

Durch Hydrierung von 10,0 g N-2-(2,4-Dimethoxyphenyl)ethyl-N-2,2,6,6-tetramethyl-4-piperidinyl-2,5-dimethylfuran-3-carbonsäureamid nach Beispiel 1A erhält man 5,90 mg schwach gelbliches, nicht destillierbares Öl.

**Beispiel 62**

Salz aus Bis-(N-1,6-hexylen-N-2,2,6,6-tetramethyl-4-piperidinyl-tetrahydrofuran-3-carbonsäureamid und Adipinsäure

Aus 9,1 g Bis-(N-1,6-hexylen-N-2,2,6,6-tetramethyl-4-piperidinyl-tetra-hydrofuran-3-carbonsäureamid und 1,14 g Adipinsäure erhält man nach Beispiel 5 9,0 g farblosen Feststoff vom Schmp. 250 bis 252° C.

**Beispiel 63**

Salz aus 2,5-Dimethyl-tetrahydrofuran-3-carbonsäure-2,2,6,6-tetramethyl-4-piperinylester und Adipinsäure

Aus 11,2 g 2,5-Dimethyl-tetrahydrofuran-3-carbonsäure-2,2,6,6-tetramethyl-4-piperidinylester und 2,95 g Adipinsäure erhält man nach Beispiel 5 14,0 g farblosen Feststoff vom Schmp. 182 bis 184° C.

**Anwendungsbeispiel**

0,25 Teile der Verbindung aus Beispiel 1 werden in 100 Teilen Polypropylen (1320 H der Fa. BASF) durch zweimaliges Extrudieren bei 220 °C eingearbeitet und zu 200 µm dicken Platten gepreßt. Nach 14-tägiger Lagerung im Dunkeln bei 25° C zeigt die Oberfläche der Platten keinen Belag.

Die so hergestellten Platten werden der Freibewitterung unterworfen. Die Alterung wird durch Messen der "CO-Zahl" nach bestimmten Zeitintervallen bestimmt. Der Beginn der Versprödung wird mechanisch ermittelt. Er entspricht etwa einer CO-Zahl von 50. Nach einem Jahr weisen die beiden getesteten Platten CO-Zahlen von 3,33 und 5,73 auf. Die Oberfläche der Platten ist klar.

Zwei in gleicher Weise hergestellte Platten, die statt der Verbindung aus Beispiel 1 0,25 Teile Chimassorb 944 enthalten, weisen nach einem Jahr CO-Zahlen von 7,22 und 11,0 auf. Ihre Oberfläche zeigt Trübung.

**Patentansprüche**

1. Tetrahydrofurancarbonsäurederivate der allgemeinen Formel I

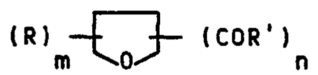 (I),

worin

R unabhängig voneinander $C_1$- bis $C_4$-Alkyl, Cyclohexyl oder Phenyl,
m 0 bis 3,
n 1 oder 2 und
R' ein Rest der Formel

$-OR^2$ , 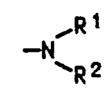 oder 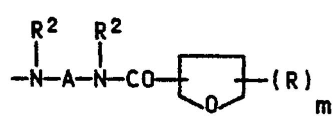

sind, worin

A $C_2$- bis $C_{12}$-Alkylen, $-(CH_2-)_2-O-(CH_2-)_2$, $-(CH_2-)_3-O-(CH_2-)_3$, $-(CH_2-)_3-O-(CH_2-)_2-O-(-CH_2-)_2$,

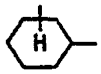 , 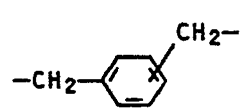 , 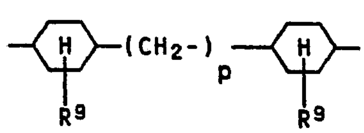

oder

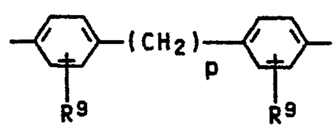

$R^1$ Wasserstoff, $C_2$- bis $C_6$-Alkenyl, gegebenenfalls bis zu 3 mal durch Sauerstoff unterbrochenes $C_1$- bis $C_{12}$-Alkyl, gegebenenfalls substituiertes Phenylalkyl oder $C_5$- bis $C_7$-Cycloalkyl und
$R^2$ ein Rest der Formel

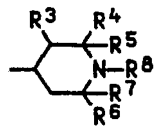

$R^3$ Wasserstoff oder Methyl,
$R^4$ bis $R^7$ Methyl oder Ethyl, und
$R^8$ Wasserstoff, $C_1$- bis $C_8$-Alkyl, $C_3$- bis $C_8$-Alkenyl, $C_2$- bis $C_4$-Hydroxyalkyl oder Aralkyl,
$R^9$ Wasserstoff oder Methyl und
p 0, 1 oder 2 bedeuten,
sowie Salze der Verbindungen (I).

2. Verbindungen gemäß Anspruch 1, wobei A eine $C_2$- bis $C_{12}$-Alkylen-gruppe,

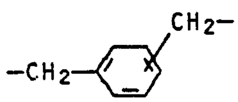 oder 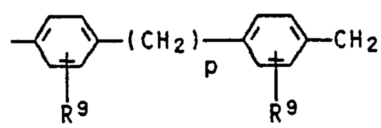

ist, worin p 1 oder 2 bedeutet.

3. Verbindungen gemäß Anspruch 1, wobei $R^3$ Wasserstoff und $R^4$ bis $R^7$ Methylgruppen sind.

4. Verbindungen gemäß Anspruch 1, wobei $R^8$ Wasserstoff oder eine Methylgruppe ist.

5. Verbindungen gemäß Anspruch 1, wobei R eine Methylgruppe ist.

6. Verwendung der Verbindungen gemäß Anspruch 1 zum Stabilisieren von organischem Material.

7. Verwendung gemäß Anspruch 6 zum Stabilisieren von Polyolefinen und Lacken.

8. Stabilisiertes organisches Material, enthaltend eine Verbindung gemäß Anspruch 1.

## Claims

1. A tetrahydrofurancarboxylic acid derivative of the formula

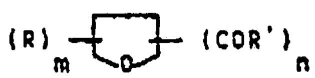

(I)

where the radicals

R independently of one another are each $C_4$-$C_4$-alkyl, cyclohexyl or phenyl,
m is from 0 to 3,
n is 1 or 2, and
R' is a radical of the formula

$-OR^2$ , 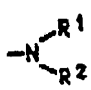 or 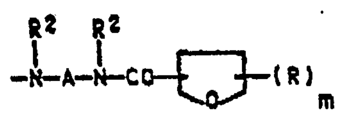

where

A is $C_2$-$C_{12}$- alkylene, $-(CH_2-)_2-O-(CH_2-)_2$, $-(CH_2-)_3-O$,$(CH_2-)_3$, $-(CH_2-)_3-O$, $(CH_2-)_2-O(CH_2-)-3$,

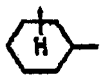 , 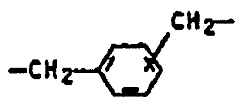 , 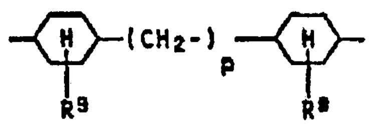

or 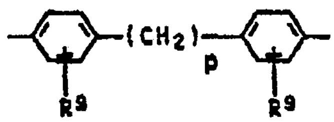

$R^1$ is hydrogen, $C_2$-$C_6$-alkenyl, $C_1$-$C_{12}$-alkyl which may be interrupted by up to 3 oxygen atoms, unsubstituted or substituted phenylalkyl or $C_5$-$C_7$-cycloalkyl and

$R^2$ is a radical of the formula

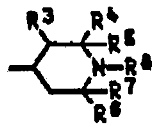

$R^3$ is hydrogen or methyl,

$R^4$, $R^5$, $R^6$ and $R^7$ are each methyl or ethyl and

$R^8$ is hydrogen, $C_1$-$C_8$-alkyl, $C_3$-$C_8$-alkenyl, $C_2$-$C_4$-hydroxyalkyl or aralkyl,

$R^9$ is hydrogen or methyl and

p is 0, 1 or 2,

and salts of the compounds (I).

2. A compound as claimed in claim 1, wherein A is a $C_2$-$C_{12}$-alkylene

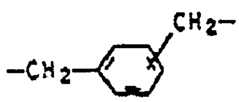 or 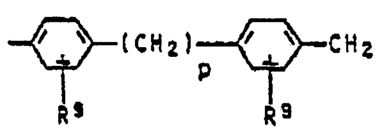

where p is 1 or 2.

3. A compound as claimed in claim 1, wherein $R^3$ is hydrogen and $R^4$, $R^5$, $R^6$ and $R^7$ are each methyl.

4. A compound as claimed in claim 1, wherein $R^8$ is hydrogen or methyl.

5. A compound as claimed in claim 1, wherein R is methyl.

6. Use of the compound as claimed in claim 1 for stabilizing organic material.

7. Use as claimed in claim 6 for stabilizing polyolefins and coatings.

8. A stabilized organic material containing a compound as claimed in claim 1.

## Revendications

1. Dérivés de l'acide tétrahydrofurannecarboxylique de formule générale I

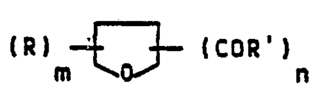 (I),

dans laquelle

R indépendamment les uns des autres sont des groupements alkyle en $C_1$-$C_4$, cyclohexyle ou phényle,

m est 0 à 3

n est 1 ou 2

R' est un reste de formule

$-OR^2$ , 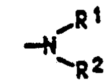 ou 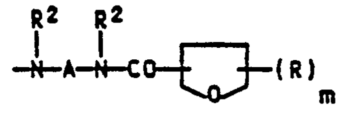

où

A représente un groupement alkylène en $C_2$-$C_{12}$, $-(CH_2-)_2-O-(CH_2-)_2$, $-(CH_2-)_3-O-(CH_2-)_3$, $-(CH_2-)_3-O-(CH_2-)_2-O-(-CH_2-)_3$,

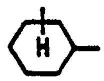 , 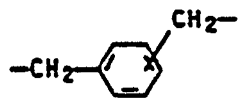 , 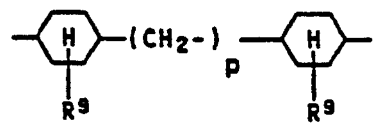

ou 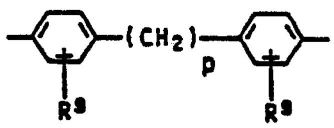

$R^1$ est un atome d'hydrogène ou un groupement alcényle en $C_2$-$C_6$, alkyle en $C_1$-$C_{12}$ éventuellement interrompu jusqu'à trois fois par de l'oxygène, phénylalkyle éventuellement substitué ou cycloalkyle en $C_5$-$C_7$,

$R^2$ est un reste de formule

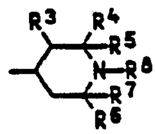

$R^3$ est un atome d'hydrogène ou un groupement méthyle,

$R^4$ à $R^7$ sont des groupements méthyle ou éthyle,

$R^8$ est un atome d'hydrogène ou un groupement alkyle en $C_1$-$C_8$, alcényle en $C_3$-$C_8$, hydroxyalkyle en $C_2$-$C_4$ ou aralkyle,

$R^9$ est un atome d'hydrogène ou un groupement méthyle, et

p est 0, 1 ou 2,

ainsi que les sels des dérivés (I),

2. Dérivés selon la revendication 1, dans laquelle A est un groupement alkylène en $C_2$-$C_{12}$,

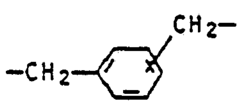 ou 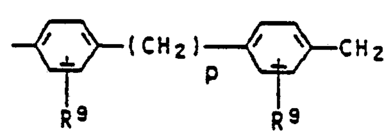

où p vaut 1 ou 2.

3. Dérivés selon la revendication 1, dans lesquels $R^3$ est un atome d'hydrogène et $R^4$ à $R^7$ sont des groupements méthyle.

4. Dérivés selon la revendication 1, dans lesquels $R^8$ est un atome d'hydrogène ou un groupement méthyle.

5. Dérivés selon la revendication 1, dans lesquels R est un groupement méthyle.

6. Utilisation des dérivés selon la revendication 1, pour la stabilisation de matières organiques.

7. Utilisation selon la revendication 6, pour la stabilisation de polyoléfines et de laques.

3. Matière organique stabilisée, contenant un dérivé selon la revendication 1.